# EUROPEAN PATENT SPECIFICATION

(11) **EP 0 885 223 B1**
(45) Date of publication and mention of the grant of the patent: **19.09.2001**
(21) Application number: 97902336.3
(22) Date of filing: 04.02.1997
(51) Int. Cl.: C07D 473/00, A61K 31/52

(54) **PHARMACEUTICAL COMPOSITIONS COMPRISING FAMCICLOVIR MONOHYDRATE**
PHARMAZEUTISCHE ZUSAMMENSETZUNGEN ENTHALTEND FAMCICLOVIR MONOHYDRAT
PREPARATIONS COMPRENANT UN MONOHYDRATE DE FAMCICLOVIR

(30) Priority: 07.02.1996 GB 9602403; 05.09.1996 GB 9618494
(43) Date of publication of application: 23.12.1998
(73) Proprietor: Novartis International Pharmaceutical Ltd., Hamilton HM 12 (BM)
(72) Inventor: CAMPBELL, Kenneth, C. - SmithKline Beecham Pharm., West Sussex BN14 8QH (GB); GREENWAY, Michael, John-SmithKline Beecham Pharm., West Sussex BN14 8QH (GB); HANCOCK, Stephen, A. - SmithKline Beecham Pharma., West Sussex BN148QH 4 (GB)
(74) Representative: Vallet, Lucien, Dr.
(86) International application number: EP9700523
(87) International publication number: WO9729108

(56) References cited:
- EP-A- 0 182 024

## Description

This invention relates to pharmaceutical compositions comprising famciclovir monohydrate.

EP-A-182024 (Beecham Group p.l.c.), Example 2 describes a method of the preparation of famciclovir, a compound which is useful as the oral form of the compound, penciclovir which has antiviral activity against infections caused by herpesviruses, such as herpes simplex type 1, herpes simplex type 2 and varicella zoster virus, and also against Hepatitis B virus. Penciclovir and its antiviral activity is disclosed in Abstract P.V11-5 p.193 of 'Abstracts of 14th Int. Congress of Microbiology', Manchester, England 7-13 September 1986 (Boyd et. al.).

The form of famciclovir used for formulating into tablets or capsules is the anhydrous form as this form is stable and has good handling qualities for commercial production. In the case of a suspension formulation, however this form of famciclovir has potential disadvantages in terms of crystal growth in solution.

Harnden et al, J. Med Chem., 1989, 32, 1738 discloses that a crystalline monohydrate deposits from concentrated aqueous solutions of famciclovir.

A pure, crystalline hydrate of famciclovir has been discovered to have surprisingly improved properties useful in a suspension formulation.

Accordingly, the present invention provides a pharmaceutical composition comprising famciclovir monohydrate, and a pharmaceutically acceptable carrier.

The hydrate is used in pharmaceutically acceptable form. By pharmaceutically acceptable form is meant, *inter alia*, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%. One preferred pharmaceutically acceptable form is the crystalline form, including such form in a pharmaceutical composition.

Famciclovir monohydrate may be prepared by a process which comprises dissolving anhydrous famciclovir in an aqueous medium and allowing the monohydrate to precipitate out from the solution.

The anhydrous famciclovir is preferably dissolved in hot water at a temperature greater than 25 degrees centigrade, usually 50 to 60 degrees centigrade, and the hot solution allowed to cool slowly to 5 degrees centigrade with continuous stirring. The monohydrate crystals are then filtered off and allowed to dry at ambient temperature.

The monohydrate may also be formed by exposing the anhydrous form of famciclovir to an atmosphere containing a high concentration of water vapour.

In particular, the invention comprises a pharmaceutical composition in the form of an aqueous suspension for oral administration.

Suspension formulations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; bulking agents such as microcystalline cellulose or silicon dioxide; flow agents such as colloidal silica; preservatives, for example methyl or propyl *p*-hydroxybenzoate or sorbic acid, and if desired conventional flavouring or colouring agents.

The oral compositions may be prepared by conventional methods.

The invention further provides the use of famciclovir monohydrate in the manufacture of a medicament for use in the treatment of viral infections.

The amount of famciclovir in the oral dosage form will depend on the viral infection being treated, the type of patient and the frequency of administration. Unit dosages are likely to be 125, 250, 500, 750 mg or lg, 1 to 3 times a day.

The invention also provides famciclovir monohydrate for use as an active therapeutic substance, in particular for use in the treatment of viral infections.

The following example illustrates the preparation of famciclovir monohydrate and the following comparison test results illustrate the beneficial use of famciclovir monohydrate in a suspension formation. European Journal of Pharmaceutical Sciences, Vol 4 Suppl., September 1996, S170, abstract P3.029, describes the use of FT-Raman Spectroscopy to characterise the pseudopolymorphic transformation of the anhydrate to the monohydrate, and AAPS 11th Annual Meeting, Seattle, WA, October 27-31, 1996, Abstract /.PDD 7140, *Pharm. Res.,* **13**, S-267, 1996, describes the compaction induced solid state reactivity of the anhydrate.

### Example

Famciclovir (150g) was dissolved in hot water (approximately 200ml at 50 to 60 degrees centigrade). The hot solution was allowed to cool slowly to 5 degrees centigrade with continuous stirring for 3 hours. The monohydrate crystals were filtered and then dried by allowing the excess water to evaporate under ambient conditions for approximately 2 days.

The monohydrate of famciclovir was characterised by infra-red spectroscopy, thermal analysis and X-ray diffraction methods. Identification was confirmed by proton nuclear magnetic resonance spectroscopy.

Water was determined at 5.3% (theoretical - 5.31%) by coulometric titration. This was confirmed by thermogravimetric analysis of the monohydrate which gave a 5.2% weight loss.

### Comparison Test Results

An investigation was carried out on monohydrate crystal growth in famciclovir suspension. Two suspensions were prepared using the formulae below and were reconstituted with water.

| Famciclovir Monohydrate Suspension | | Famciclovir Anhydrate Suspension | |
|---|---|---|---|
| | % Composition | | % Composition |
| Famciclovir Monohydrate | 35.20 | Famciclovir Anhydrate granules | 34.36 |
| Hydroxy Propyl Methyl Cellulose | 3.33 | Hydroxy Propyl Methyl Cellulose | 3.33 |
| Xanthan Gum | 3.33 | Xanthan Gum | 3.33 |
| Saccharin | 1.78 | Saccharin | 1.78 |
| Aspartame | 2.67 | Aspartame | 2.67 |
| Colloidal Silica | 1.67 | Colloidal Silica | 1.67 |
| Flavours | 6.93 | Flavours | 6.93 |
| Disodium hydrogen Phosphate dihydrate | 19.56 | Disodium hydrogen Phosphate dihydrate | 15.6 |
| Citric acid monohydrate | 2.7 | Citric acid monohydrate | 2.47 |
| Silicon Dioxide | 22.38 | Silicon Dioxide | 27.41 |

The reconstituted suspension was stored at 25°C and the crystal growth monitored over a period of one week using microscopy.

The results from visual and photographic examination indicated little or no crystal growth in the monohydrate suspension whilst the crystals in the anhydrate suspension had grown to ten times their original size, making them less pharmaceutically acceptable.

## Claims

1. A pharmaceutical composition comprising famciclovir monohydrate, and a pharmaceutically acceptable carrier.

2. A pharmaceutical composition according to claim 1, in the form of an aqueous suspension for oral administration.

3. Famciclovir monohydrate for use as an active therapeutic substance.

4. Famciclovir monohydrate for use in the treatment of viral infections.

5. The use of famciclovir monohydrate in the manufacture of a medicament for use in the treatment of viral infections.

## Patentansprüche

1. Pharmazeutische Zusammensetzung, die Famciclovirmonohydrat und einen pharmazeutisch annehmbaren Träger enthält.

2. Pharmazeutische Zusammensetzung nach Anspruch 1 in Form einer wäßrigen Suspension zur oralen Verabreichung.

3. Famciclovirmonohydrat zur Verwendung als therapeutischer Wirkstoff.

4. Famciclovirmonohydrat zur Verwendung bei der Behandlung von viralen Infektionen.

5. Verwendung von Famciclovirmonohydrat zur Herstellung eines Arzneimittels zur Verwendung bei der Behandlung von viralen Infektionen.

## Revendications

1. Une composition pharmaceutique comprenant du monohydrate de famciclovir et un véhicule pharmaceutiquement acceptable.

2. Une composition pharmaceutique selon la revendication 1, sous forme d'une suspension aqueuse pour l'administration orale.

3. Le monohydrate de famciclovir pour une utilisation comme substance thérapeutique active.

4. Le monohydrate de famciclovir pour une utilisation dans le traitement d'infections virales.

5. L'utilisation du monohydrate de famciclovir pour la fabrication d'un médicament pour l'utilisation dans le traitement d'infections virales.
